**Europäisches Patentamt**

**European Patent Office**

**Office européen des brevets**

(19)

(11) Veröffentlichungsnummer: **0 007 355**
**B1**

(12) # EUROPÄISCHE PATENTSCHRIFT

(45) Veröffentlichungstag der Patentschrift: **11.05.83**

(51) Int. Cl.³: **C 02 F 1/46, A 61 L 2/02**

(21) Anmeldenummer: **78900117.9**

(22) Anmeldetag: **14.09.78**

(86) Internationale Anmeldenummer:
**PCT/DE78/00024**

(87) Internationale Veröffentlichungsnummer:
**WO 79/00145 22.03.79 Gazette 79/6**

(54) **VERFAHREN ZUR ENTKEIMUNG VON FLÜSSIGKEITEN UND VORRICHTUNG ZUR DURCHFÜHRUNG DES VERFAHRENS.**

(30) Priorität: **14.09.77 DE 2741317**

(43) Veröffentlichungstag der Anmeldung:
**06.02.80 Patentblatt 80/3**

(45) Bekanntmachung des Hinweises auf die Patenterteilung:
**11.05.83 Patentblatt 83/19**

(84) Benannte Vertragsstaaten:
**CH DE FR GB LU SE**

(56) Entgegenhaltungen:
**CH - A - 334 616**
**CH - A - 400 455**
**FR - A - 433 857**
**FR - A - 704 257**
**GB - A - 150 318**
**US - A - 2 217 895**

(73) Patentinhaber: **Reis, August K., Prof. Dr. med.**
**Faistenbergerstrasse 1**
**D-8000 München 90 (DE)**

(72) Erfinder: **Reis, August, K., Dr.**
**Faistenbergerstrasse 1**
**D-8000 München 90 (DE)**
Erfinder: **Kirmaier, Norbert L., Dr.**
**Mondstrasse 6**
**D-8011 Aschheim (DE)**
Erfinder: **Determann, Helmut, Dr.**
**Almeidaweg 19**
**D-8130 Starnberg (DE)**
Erfinder: **Thiery, Joachim, Dr.**
**Dornheimer Ring 6**
**D-6800 Mannheim 31 (DE)**
Erfinder: **Haker, Rolf**
**Dietrich-Bonnhoeffer-Strasse 27**
**D-6710 Frankenthal (DE)**
Erfinder: **Krüger, Dietrich, Dr.**
**Schubertstr. 25**
**D-6905 Schriesheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Verfahren zur Entkeimung von Flüssigkeiten und Vorrichtung zur Durchführung des Verfahrens

Die Erfindung betrifft ein Verfahren zur Entkeimung von eine sehr geringe Leitfähigkeit aufweisenden Flüssigkeiten mittels anodischer Oxidation und eine Vorrichtung zur Durchführung des Verfahrens.

Die Erfindung soll insbesondere Anwendung bei der Entkeimung von Flüssigkeiten zur Verwendung in der Arzneimittelindustrie und im medizinischen Bereich finden.

Bei der Verwendung von destilliertem Wasser oder entmineralisiertem Wasser im medizinischen Bereich bzw. in der Arzneimittelindustrie wird dieses Wasser von einer zentralen Versorgungsstelle eines Betriebes oder Krankenhauses über eine entsprechende Leitung zu dem jeweiligen Verwendungsort geliefert. Dabei besteht die Gefahr, daß das Wasser reinfiziert wird.

Am eigentlichen Einsatzort ist daher eine zusätzliche Entkeimung erforderlich. Diese erfolgt in bekannter Weise beispielsweise durch Erhitzen, wobei allerdings ein anschließendes Abkühlen erforderlich ist. Auch die Entkeimung mittels ionisierender Strahlen ist bekannt. Zur Entkeimung mittels Bestrahlung ist allerdings eine aufwendige Apparatur erforderlich. Auch eine Entkeimung mittels Filter wurde versucht, wobei jedoch das Problem auftrat, daß eine vollständige Entkeimung nur sehr schwierig zu erreichen ist.

Beispielsweise aus der DE—A—23 24 795 ist ein Verfahren zur Entkeimung mittels anodischer Oxidation und eine Vorrichtung zur Durchführung dieses Verfahrens bekannt. Damit die anodische Oxidation durchgeführt werden kann, muß die Flüssigkeit eine ausreichende Leitfähigkeit besitzen, da der Leistungsverbrauch sonst so groß wird, daß das Verfahren aus wirtschaftlichen Gründen nicht mehr eingesetzt werden kann. Zwar ist es bekannt, bei der anodischen Oxidation der Flüssigkeit beispielsweise NaCl zuzusetzen und so die Leitfähigkeit zu erhöhen und damit den erforderlichen Energieverbrauch herabzusetzen. Die Zugabe von Fremdstoffen ist jedoch bei bestimmten für im pharmazeutischen Bereich bzw. medizinischen Bereich zu verwendende Flüssigkeiten nicht erlaubt.

Durch die britische Patentschrift GB—A—150 318 ist es auch bekannt, Gase in die zu behandelnden Flüssigkieten einzuführen.

Aufgabe der Erfindung ist es, ein Verfahren zum Entkeimen von eine sehr geringe Leitfähigkeit aufweisenden Flüssigkeiten mittels anodischer Oxidation zu schaffen, welches insbesondere am Einsatzort zur Entkeimung von entmineralisiertem oder destilliertem Wasser im pharmazeutischen oder medizinischen Bereich verwendet werden kann. Ferner soll eine Vorrichtung zur Durchführung dieses Verfahrens angegeben werden.

Gemäß einer Weiterführung der Erfindung soll die Vorrichtung so ausgebildet sein, daß zum Einstellen einer vorgegebenen Leitfähigkeit bei der anodischen Oxidation eine Regeleinrichtung vorgesehen ist.

Diese Aufgabe wird durch ein Verfahren zur Entkeimung von Flüssigkeiten mittels anodischer Oxidation in Anwesenheit eines Gases gelöst, welches gemäß der Erfindung dadurch gekennzeichnet ist, daß Flüssigkeiten mit einer sehr geringen Leitfähigkeit z.B. etwa 4 $\mu$s/cm, ein ihre Leitfähigkeit erhöhendes Gas zugeführt wird und anschließend die anodische Oxidation erfolgt. Auf diese Weise ist die anodische Oxidation mit geringem Energieeinsatz und ohne Zusatz von chemisch lösbaren Fremdstoffen möglich.

Die Vorrichtung zur Durchführung des Verfahrens enthält einen mit Elektroden bestückten Oxidationsreaktor und ist gemäß der Erfindung dadurch gekennzeichnet daß der Oxidationsreaktor außer einem Eingang und einem Ausgang ein eingangsseitig angeordnetes Zuführungsmittel zur Zuführung eines Gases und eine ausgangsseitig vorgesehenen Belüftungsmöglichkeit aufweist.

Gemäß einer Weiterbildung der Erfindung ist das Verfahren dadurch gekennzeichnet, daß das zugeführte Gas nach der anodischen Oxidation aus der Flüssigkeit entfernt wird.

Bei einer Weiterbildung der Erfindung wird das Gas mittels Inertgasbelüftung entfernt. Auf diese Weise wird ein besonders schnelles und vollständiges Entfernen des zugeführten Gases erreicht.

Eine Weiterbildung der Erfindung ist dadurch gekennzeichnet, daß zur Erhöhung des Anteils des zuzuführenden Gases der Druck in der mittels anodischer Oxidation zu entkeimenden Flüssigkeit während des Zuführens dieses Gases bis zur Beendigung der anodischen Oxidation erhöht wird. Durch die dadurch erzielte größere Aufnahme des Gases in der Flüssigkeit wird die Leitfähigkeit noch weiter erhöht.

Gemäß einer Weiterbildung der Erfindung ist die Vorrichtung dadurch gekennzeichnet, daß eingangsseitig von dem Oxidationsreaktor ein Bereich großen Fließquerschnitts vorgesehen ist und daß das Gas in diesen Bereich einleitbar ist. Auf diese Weise wird die Wechselwirkung der Flüssigkeit mit dem eingeführten Gas und damit die Lösung desselben in der Flüssigkeit vergrößert.

Die Vorrichtung weist am Eingang der Zuführungsmittel in den Bereich großen Fließquerschnitts ein das eintretende Gas fein verteilendes Mittel auf. Dadurch wird eine größere Löslichkeit des Gases und somit eine höhere Leitfähigkeit erreicht.

Eine besondere Ausführungsform der Erfindung ist dadurch gekennzeichnet, daß ausgangsseitig vor der Belüftungsmöglichkeit eine Drosselstelle vorgesehen ist. Auf diese Weise steht die Flüssigkeit in dem Oxidationsreaktor unter Druck, wodurch eine größere Menge Gases in der Flüssigkeit lösbar ist.

Eine Weiterbildung der Erfindung ist dadurch gekennzeichnet, daß die Belüftungsmöglichkeit einen Bereich größeren Fließquerschnitts mit einem Gasaustritt aufweist und ein Zuführungsmittel zur Einleitung eines inerten Gases in diesen Bereich vorgesehen ist, wodurch erreicht wird, daß das eingangsseitig gelöste Gas vollständig aus der Flüssigkeit herausgetrieben wird.

Weitere Merkmale und Zweckmäßigkeiten der Erfindung ergeben sich aus der Beschreibung von Ausführungsbeispielen anhand der Figuren. Von den Figuren zeigen:

Fig. 1 eine schematische Darstellung der Vorrichtung bzw. Anordnung gemäß der Erfindung; und

Fig. 2 eine Weiterbildung der Erfindung.

In den Figuren ist der Oxidationsreaktor mit 1 bezeichnet. Er kann vorzugsweise als Stabgitterbündelzelle ausgebildet sein.

In dem Oxidationsreaktor wird eingangsseitig das zu entkeimende Fluid, insbesondere entmineralisiertes Wasser bzw. destilliertes Wasser, über eine Eingangsleitung 2 zugeführt. Vor dem Oxidationsreaktor ist ein Gasmischbehälter 3 vorgesehen, durch den die Flüssigkeit hindurchtritt. Auf der Unterseite des Gasmischbehälters ist eine mit einem $CO_2$-Vorratsbehälter 4 verbundene Zuführungsleitung 5 vorgesehen. In einem Abstand vom Boden 6 des Gasmischbehälters ist eine den ganzen Querschnitt des Gasmischbehälters ausfüllende poröse Schicht 7 vorgesehen. In dem Gasmischbehälter ist ein Rührer 8 zum Umrühren der durchlaufenden Flüssigkeit angeordnet. Auf der Oberseite weist der Gasmischbehälter eine Öffnung 9 auf, durch die Gas entweichen kann. In der Zuführungsleitung 5 sind ein Drosselventil 10 und eine Pumpe 11 vorgesehen, mittels der das Gas aus dem Vorratsbehälter 4 mit vorgewähltem Druck in den Gasmischbehälter einführbar ist. Anstelle der porösen Schicht können auch perforierte Rohre, Schläuche, oder Siebböden oder sonstige Mittel verwendet werden, die eine feine Verteilung des Gases in der Flüssigkeit gewährleisten.

An der Ausgangsseite des Oxidationsreaktors ist eine Ausgangsleitung 12 vorgesehen, über die das entkeimte Fluid zu dem Verbraucher geführt wird. Hinter dem Oxidationsreaktor ist ein Gasentmischungsbehälter 13 vorgesehen. In dessen Boden mündet eine mit einem $N_2$-Vorratsbehälter 14 verbundene Leitung 15. In der Leitung 15 sind zwischen dem Vorratsbehälter 14 und dem Gasentmischungsbehälter 13 eine Pumpe 16 und ein Drosselventil 17 zum Einstellen des Druckes des über die Leitung 15 zuzuführenden Gases vorgesehen. Ferner ist vor dem Eintritt in den Gasentmischungsbehälter ein Inertgassterilisationsfilter 18 zur Gassterilisation angeordnet.

In einem geringen Abstand vom Boden des Gasentmischungsbehälters 13 ist eine den gesamten Behälterquerschnitt bedeckende poröse Schicht 19 vorgesehen. Am Boden des Gasentmischungsbehälters ist eine Vibrationseinrichtung 20 vorgesehen, die mittels einer elektronischen Steuerung 21 einstellbar ist. An der Oberseite des Gasentmischungsbehälters ist ein Belüftungsrohr 22 zum Gasaustritt vorgesehen. Gewünschtenfalls kann anstelle des Rohres auch eine Öffnung in der Abdeckung des Behälters vorgesehen sein. Auch hier können anstelle der porösen Schicht andere bekannte Mittel verwendet werden, die eine feine Verteilung des Gases in der Flüssigkeit ermöglichen.

Mit der oben beschriebenen Vorrichtung wird das Verfahren so durchgeführt, daß die über die Eingangsleitung 2 zugeführte Flüssigkeit in den Gasmischbehälter eintritt. In den Behälter wird über die Zuführungsleitung 5 von der Bodenseite her $CO_2$ zugeführt. Diese wird beim Hindurchtreten durch die poröse Schicht 7 fein über den ganzen Behälterquerschnitt verteilt, so daß eine grosse Wechselwirkung und damit eine sehr starke Lösung im Wasser erfolgt. Die Lösung des $CO_2$ wird noch durch Betätigen des Rührers 8 verstärkt. Überschüssiges nichtgelöstes $CO_2$ kann durch die Öffnung 9 nach oben austreten.

Die Flüssigkeit, die nun eine ausreichende Leitfähigkeit besitzt, fließt über eine Verbindungsleitung 23 in den Oxidationsreaktor, wo die eigentliche Entkeimung stattfindet. Die aus dem Oxidationsreaktor austretende Flüssigkeit gelangt dann in den Gasentmischungsbehälter. In diesen wird vom Boden her $N_2$-Gas zugeführt, welches durch die poröse Schicht fein verteilt in das Wasser eintritt und das gelöste $CO_2$ vollständig austreibt. Die Größe des Gasentmischungsbehälters bestimmt die Verweildauer der Flüssigkeit in diesem Behälter. Der Behälter wird so gewählt, daß bei vorgegebenem Druck des zuzuführenden $N_2$-Gases das gelöste $CO_2$ ausgetrieben wird. Umgekehrt wird bei vorgegebener Behältergröße der Druck des $N_2$ so gewählt, daß das $CO_2$-Gas ausreichend ausgetrieben wird. Über die Ausgangsleitung 12 wird das nun vollständig entkeimte und von allen Fremdstoffen freie Wasser der Verbrauchsstelle zugeführt.

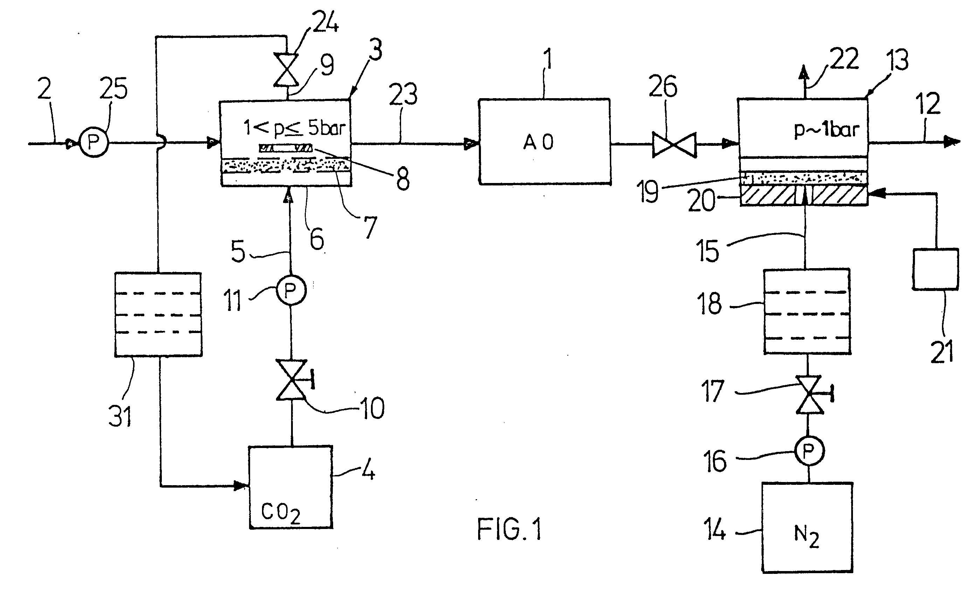

Die Aufgabe der Erfindung wird optimal bei einer Ausführungsform gelöst, bei der die Flüssigkeit im Oxidationsreaktor und im Gasmischbehälter unter einem Druck von vorzugsweise mehr als 1 bis 5 bar steht. Bei dieser Ausführungsform, die in der Figur 1 dargestellt ist, ist die Öffnung 9 als ein Rohr ausgebildet, welches über ein Drosselventil 24 und eine Gasreinigungsvorrichtung 31 zu dem $CO_2$-Vorratsbehälter 4 zurückführt. Ferner ist auf der Eingangsseite vor dem Gasmischbehälter 3 eine Pumpe 25 vorgesehen. An der Ausgangsseite des Oxidationsreaktors 1 ist vor dem Gasentmischungsbehälter 13 eine Drosselstelle 26 vorgesehen, in der eine Druckentspannung erfolgt.

Die Vorrichtung arbeitet so, daß mit der Pumpe 25 ein vorgegebener Wasserdruck von mehr als 1 bar und bis zu etwa 5 bar erzeugt wird. Der Druck in der Zuführungsleitung 5 wird entsprechend größer eingestellt, so daß eine ausreichende Lösung des $CO_2$ erfolgt. Über die Öffnung 9 entweichendes $CO_2$ wird in einem Kreislauf dem $CO_2$-Vorratsbehälter zurückgeführt. Nach Durchführen der anodischen Oxidation im Oxidationsreaktor erfolgt eine Druckentlastung mittels des Drosselventils 26, wodurch schon ohne weitere äußere Einwirkung ein beachtlicher Anteil des gelösten $CO_2$ aus der Lösung frei wird. Durch die Einwirkung des unter Druck zugeführten $N_2$-Gases wird das $CO_2$ schließlich vollständig aus dem Wasser ausgetrieben.

Mit dem oben beschriebenen Verfahren wurde bei destilliertem Wasser die aus der Tabelle 1 ersichtliche Leitfähigkeit erzielt. Die Tabelle gibt die Leitfähigkeit ohne bzw. mit zugeführtem $CO_2$ wieder, wobei $CO_2$ im letzteren Fall bis zur Sättigung zugeführt wurde. In den letzten drei Zeilen sind die Werte angegeben, die gemessen wurden, nachdem das $CO_2$ mit Hilfe von $N_2$ über eine, zwei und fünf Minuten ausgetrieben worden ist.

### TABELLE 1

| $CO_2$-Eintrag in destilliertes Wasser mit anschließender anschließender $N_2$-Ausblasung | | | | | |
|---|---|---|---|---|---|
| Wasserart bzw. -Zustand | Temperatur T (°C) | Leitfähigkeit ($\mu$S/cm) | pH-Wert (—) vor $CO_2$- Zuführung | $CO_2$-Konzentration (mmol/l) | Redox-Potential (mV) |
| Aqua dest. ohne $CO_2$ (an der Luft gelagert) | 22 | 4 | 5,3 | 0,3 | 302 |
| Aqua dest. mit $CO_2$ | 21 | 37 | 4,25 | 10 | 390 |
| 1 min Ausblasen mit $N_2$ | 20 | 11 | 4,93 | 0,8 | 378 |
| 2 min Ausblasen mit $N_2$ | 20 | 5 | 6,08 | 0,2 | 328 |
| 5 min Ausblasen mit $N_2$ | 19,5 | 3 | 6,64 | 0,1 | 308 |

Bezüglich der Energieeinsparung ergaben Versuche folgendes: Durch Zuführen von $CO_2$ wurde die Leitfähigkeit des in den Oxidationsreaktor eintretenden Wassers auf 38 $\mu$S/cm eingestellt. Zum Erreichen einer Stromdichte im Oxidationsreaktor von 2 mA/cm² wurde ein Strom von 200 mA eingestellt. Die anzulegende Zellspannung betrug dann 290 V. Die rechnerische Auswertung ergab, daß bei einer Leitfähigkeit ohne $CO_2$-Zusatz von 2 $\mu$S/cm bei der gleichen Stromdichte im Oxidationsreaktor eine Spannung von 4.640 V und bei einer Leitfähigkeit von 1 $\mu$S/cm bei gleicher Stromdichte 9.280 V erforderlich sein würden. Die letzten beiden Werte wurden errechnet, da experimentell bei Anlegen dieser Spannungen kein einwandfreies Funktionieren möglich war sondern ein Stromdurchschlag erfolgte. Bei diesem Versuch war als Flüssigkeit destilliertes Wasser verwendet worden.

Bei Verwendung entmineralisierten Wassers ergab ein entsprechender Versuch bei einer

Leitfähigkeit von 45 $\mu$S/cm und der gleichen Stromdichte eine anzulegende Spannung von 240 V. Würde man dagegen kein $CO_2$ zugeführt haben, hätte die Leitfähigkeit im Bereich von 1 bis 2 $\mu$S/cm betragen. Bei 2 $\mu$S/cm wären 5.400 V und bei 1 $\mu$S/cm 10.800 V Zellspannung erforderlich gewesen.

Aus dem ersten Versuch ergibt sich, daß die aufzuwendende Leistung mit $CO_2$ 58 W und ohne $CO_2$ 1.856 W beträgt. Durch den erfindungsgemäßen Zusatz von $CO_2$ ist also nur 1/32 der Leistung erforderlich, die ohne den Zusatz von $CO_2$ aufzuwenden wäre. Bei dem zweiten Versuch mit entmineralisiertem Wasser ist bei Zusatz von $CO_2$ eine Leistung von 48 W und ohne den Zusatz dieses Gases eine Leistung von 2.160 W erforderlich. Das bedeutet, daß die aufzuwendende Leistung ohne den Zusatz von $CO_2$ mal größer als die bei Zusatz von $CO_2$ ist.

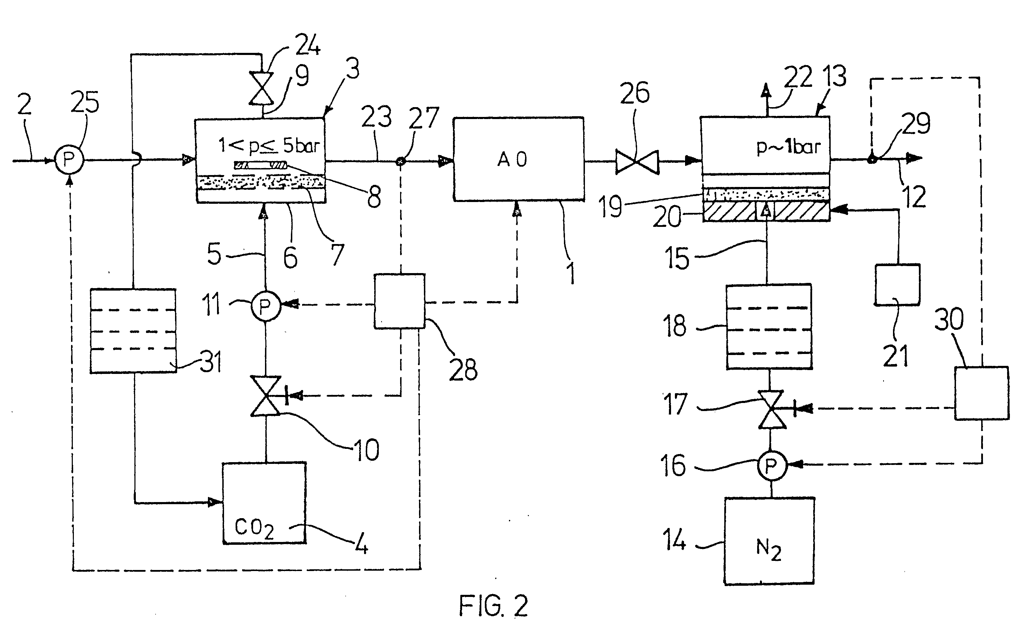

In Figur 2 ist eine Ausführungsform der Erfindung beschrieben, die im Grundaufbau mit der oben beschriebenen Vorrichtung übereinstimmt und bei der die gleichen Teile wie in Figur 1 mit den gleichen Bezugszeichen bezeichnet sind. In dieser Ausführungsorm ist zwischen dem Gasmischbehälter und dem Oxidationsreaktor ein Meßfühler 27 angeordnet, der den $CO_2$-Gehalt mißt und ein Ausgangssignal an einen Regler 28 gibt. In Abhängigkeit von der Abweichung des gemessenen $CO_2$-Gehaltes von dem Sollwert steuert der Regler mit seinem Ausgangssignal die $CO_2$-Zufuhr und den Druck im Gasmischbehälter bzw. Oxidationsreaktor. Anstelle des $CO_2$-Gehaltes kann mit dem Meßfühler auch die Leitfähigkeit bzw. der pH-Wert gemessen werden. Der Meßfühler könnte auch eine Kombination dieser Werte erfassen. Sobald sich aus dem Meßwert ergibt, daß auch bei Nachstellen der $CO_2$-Zufuhr und Nachstellen der Druckerhöhung die Anodenspannung nicht ausreicht, kann der Regler über ein Ausgangssignal die an dem Oxidationsreaktor 1 anliegende Zellspannung oder den Strom nachregeln. Auf diese Weise wird erreicht, daß die Apparatur automatisch arbeiten kann.

In Figur 2 ist an der Ausgangsseite des Gasentmischungsbehälters 13 ein Meßfühler 29 vorgesehen, der die Konzentration des im Gasmischungsbehälter zugeführten Gases mißt. Das Ausgangssignal des Meßfühlers wird einem Regler 30 zugeführt, der mit seinem Ausgangssignal die Zufuhr des $N_2$-Gases und dessen Druck so regelt, daß der Gehalt des im Gasmischbehälter 3 zugeführten Gases an der Ausgangsseite des Gasentmischungsbehälters 13 nicht übersteigt.

In den oben beschriebenen Ausführungsbeispielen wurde zur Erhöhung der Leitfähigkeit $CO_2$ verwendet. Grundsätzlich können auch andere die Leitfähigkeit erhöhende Gase verwendet werden, wie beispielsweise $F_2$, wenn der Verwendungszweck des zu erzeugenden Wassers dies zuläßt. Das $CO_2$ ist jedoch deshalb allen anderen Gasen überlegen, weil es weder toxisch ist noch explosiv und sich andererseits nach der anodischen Oxidation wieder leicht austreiben läßt.

Das Austreiben erfolgte in dem oben beschriebenen Aufführungsbeispiel mittels $N_2$-Gas. Anstelle des $N_2$-Gases können auch Edelgase, insbesondere Heliumgas verwendet werden.

## Patentansprüche

1. Verfahren zur Entkeimung von Flüssigkeiten mittels anodischer Oxidation in Anwesenheit eines Gases, dadurch gekennzeichnet, daß Flüssigkeiten mit einer geringen Leitfähigkeit, z.B. etwa 4 $\mu S/cm$, ein die Leitfähigkeit erhöhtes Gas zugeführt wird und anschließend die anodische Oxidation erfolgt.

2. Verfahren nach Anspruch 1, dadurch gekennzeichnet, daß das zugeführte Gas nach der anodischen Oxidation aus der Flüssigkeit entfernt wird.

3. Verfahren nach Anspruch 2, dadurch gekennzeichnet, daß die Entfernung mittels einer Inertgasbelüftung erfolgt.

4. Verfahren nach einem der Ansprüche 1 bis 3, dadurch gekennzeichnet, daß zur Erhöhung des Anteils des gelösten Gases der Druck in der mittels anodische Oxidation zu entkeimenden Flüssigkeit während des Zuführens dieses Gases bis zur Beendigung der anodischen Oxidation erhöht wird.

5. Verfahren nach einem der Ansprüche 1 bis 4, dadurch gekennzeichnet, daß das zuzuführende Gas $CO_2$ ist.

6. Vorrichtung zur Durchführung des Verfahrens nach einem der Ansprüche 1—5 in einem mit Elektroden bestückten Oxidationsreaktor, dadurch gekennzeichnet, daß der Oxidationsreaktor (1) außer einem Eingang und einem Ausgang ein eingangsseitig angeordnetes Zuführungsmittel (5) zur Zuführung eines Gases und eine ausgangsseitig vorgesehene Belüftungsmöglichkeit (22) aufweist.

7. Vorrichtung nach Anspruch 6, dadurch gekennzeichnet, daß eingangsseitig ein Bereich (3) großen Fließquerschnitts vorgesehen ist und daß Gas in diesen Bereich einleitbar ist.

8. Vorrichtung nach Anspruch 7, dadurch gekennzeichnet, daß am Eingang der Zuführungsmittel in den Bereich (3) ein das eintretende Gas fein verteilendes Mittel (7) vorgesehen ist.

9. Vorrichtung nach einem der Ansprüche 6 bis 8, dadurch gekennzeichnet, daß ausgangsseitig vor der Belüftungsmöglichkeit (22) eine Drosselstelle (26) vorgesehen ist.

10. Vorrichtung nach einem der Ansprüche 7 bis 9, dadurch gekennzeichnet, daß die Belüftungsmöglichkeit (22) einen Bereich (13) größeren Fließquerschnitts mit einem Gasaustritt aufweist und ein Zuführungsmittel (15) zur Einleitung eines inerten Gases in diesen Bereich vorgesehen ist.

## Revendications

1. Procédé de stérilisation de liquides par oxidation anodique en présence d'un gaz, caractérisé par le fait qu'un gaz est ajouté aux liquides présentant une faible conductivité, par. ex. 4 $\mu S/cm$ env., qui augmente la conductivité de ces liquides; ce n'est qu'ensuite que l'oxidation anodique a lieu.

2. Procédé selon revendication 1, caractérisé par le fait que le gaz ajouté est éliminé du liquide après l'oxidation anodique.

3. Procédé selon revendication 2, caractérisé par le fait que l'élimination du gaz se fait par aération par gaz inerte.

4. Procédé selon une des revendications 1 à 3, caractérisé par le fait que pour augmenter la part du gaz dissout, la pression du liquide qui doit être stérilisé par oxidation anodique augmente pendant l'apport du gaz jusqu'à la fin de l'oxidation anodique.

5. Procédé selon une des revendications 1 à 4, caractérisé par le fait que le gaz ajouté est du $CO_2$.

6. Dispositif pour l'application du procédé selon une des revendications 1 à 5 dans un réacteur d'oxidation muni d'électrodes, caractérisé par le fait que le réacteur d'oxidation (1) présente, en plus d'une entrée et d'une sortie, une amenée (5) disposée côté entrée pour l'entrée d'un gaz et un moyen d'aération (22) côté sortie.

7. Dispositif selon revendication 6, caractérisé par le fait qu'une zone (3) présentant une large section est prévue et que le gaz peut être amené dans cette zone.

8. Dispositif selon revendication 7, caractérisé par le fait qu'un dispositif (7) est prévu à l'entrée de l'amenée dans la zone (3) pour disperser le gaz d'apport.

9. Dispositif selon une des revendications 6 à 8, caractérisé par le fait qu'une soupape d'étranglement (26) est prévue côté sortie devant le moyen d'aération (22).

10. Dispositif selon une des revendications 7 à 9, caractérisé par le fait que le moyen d'aération (22) présente une zone (13) avec une section élargie, une sortie de gaz ainsi qu'une amenée (15) pour l'apport du gaz inerte dans cette zone.

## Claims

1. A method for degerminating liquids by means of anodic oxidation in the presence of a gas, characterized by the fact that a gas which increases the conducting capacity is added to liquids having little conducting capacity, e.g. approx. 4 $\mu$S/cm, and subsequently anodic oxidation takes place.

2. A method according to claim 1, characterized by the fact that the added gas is removed from the liquid once the anodic oxidation has taken place.

3. A method according to claim 2, characterized by the fact that the separation of the gas is carried out by ventilating with inert gas.

4. A method according to one of the claims 1 to 3, characterized by the fact that the pressure of the liquid which is to be degerminated by means of anodic oxidation is increased while the solute gas is added until the anodic oxidation has been completed.

5. A method according to one of the claims 1 to 4, characterized by the fact that the gas to be added is $CO_2$.

6. A device for carrying out the method according to one of claims 1—5 in an oxidation reactor equipped with electrodes, characterized by the fact that the oxidation reactor (1) in addition to an entrance and an exit is provided with a feeding means (5) situated at the side of the entrance for admitting the gas and with a ventilation means (22) at the side of the exit.

7. A device according to claim 6, characterized by the fact that a zone (3) with a large flowing cross-section is provided at the entrance side and that gas can be passed into this zone.

8. A device according to claim 7, characterized by the fact that an appliance (7) has been provided at the entrance of the feeding means in order to distribute the entering gas finely.

9. A device according to one of the claims 6 to 8, characterized by the fact that a reduction point (26) has been provided at the exit side prior to the ventilation possibility (22).

10. A device according to one of the claims 7 to 9, characterized by the fact that the ventilation possibility (22) has a zone (13) with a large flowing cross-section and a gas exit, and that a charging means (15) for introducing inert gas into this zone has been provided.

FIG.1

FIG. 2

0 007 355